# EUROPEAN PATENT APPLICATION

(11) **EP 4 458 763 A1**
(43) Date of publication of application: **06.11.2024**
(21) Application number: 23315166.1
(22) Date of filing: 05.05.2023
(51) Int. Cl.: C01B 3/02, B01D 59/44, G01N 33/22

(54) **METHOD FOR CERTIFYING ORIGIN OF A HYDROGEN-BASED FLOW**

(71) Applicant: TOTALENERGIES ONETECH, 92400 Courbevoie (FR)
(72) Inventor: Dubouis, Nicolas, 92078 PARIS LA DEFENSE (FR); Ayme-Perrot, David, 92078 PARIS LA DEFENSE (FR)
(74) Representative: Lavoix

(57) **Abstract**

The invention relates to a method (100) for certifying origin of a purchased hydrogen-based flow, comprising the following steps:
- generating a flow of dihydrogen (23) from an initial flow (22) ;
- converting said flow of dihydrogen (23) into a produced hydrogen-based flow (25) by chemical reaction with a reacting agent from a reagent flow (24), said produced hydrogen-based flow (25) comprising H₂ derivatives.

The method comprises the following preliminary steps of:
- measuring, in samples of the initial flow (22) and of the reagent flow (23), ratios of stable isotopes of at least one element, to obtain feedstock isotope ratios ;
- associating each feedstock isotope ratio with a unique identifier to form identification information.

The method further comprises a preliminary step of acquiring partition coefficients specific to the generating step and to the converting step.

## Description

### FIELD OF THE INVENTION

The present invention concerns the field of clean energy, and more particularly a method for certifying the origin of a production of low-carbon dihydrogen derivatives. The present invention particularly relates to a method for certifying the origin of a hydrogen-based flow, as well as a computer program product and a system for carrying out such method.

### BACKGROUND OF THE INVENTION

Hydrogen can be produced from various energy sources, and the cost, amount, and energy-footprint of hydrogen vary, depending on production methods. The type of method used for forming the hydrogen greatly affects the environmental value (CO2 emission in production, transportation, and the like) and economic value of hydrogen.

In this connection, a certification project have been launched in Europe in efforts for establishing a hydrogen trading market. In this project, the environmental value of hydrogen is defined in three categories, that is, Green hydrogen, Low Carbon hydrogen, and Gray hydrogen, based on CO₂ emission intensity. Gray hydrogen is, for example, natural gas reformed hydrogen, which is produced from a fossil fuel as a raw material. Its CO₂ emission intensity is therefore high, because of which its environmental value is evaluated to be low. Green hydrogen (e.g., hydrogen produced by electrolyzing water using electricity generated by renewable power generation, such as wind power generation or solar power generation) and Low Carbon hydrogen (e.g., hydrogen produced by electrolyzing water using electricity from power generation systems), on the other hand, show low CO₂ emission intensity, and are therefore evaluated to be low in environmental value.

The demand for green hydrogen or low-carbon hydrogen in the industry is increasing worldwide. However, from a molecular point of view, a green dihydrogen molecule or low-carbon dihydrogen molecule are the same as gray dihydrogen molecule, so that the origin of hydrogen cannot easily be determined.

Certification of origin based on digital methods are employed for tracking green electricity and potentially green hydrogen and downstream products, for example by issuing certificate following a regular audit of the production chain, the certificate being handed to the hydrogen purchaser at the time of the hydrogen delivery or purchase.

However, these techniques are not entirely satisfying and they need improvement to guarantee transparency and reliability in the certification of origin of hydrogen or hydrogen derivatives.

### SUMMARY OF THE INVENTION

One aim of the present invention is to improve the existing techniques and to make it possible to authenticate the origin of hydrogen-based flow in a simple and reliable way, without negatively affecting the quality of the hydrogen-based flow.

To that end, the present invention relates to a method for certifying origin of a purchased hydrogen-based flow, comprising the following steps:
- generating, notably by water electrolysis or natural gas reforming, a flow of dihydrogen from an initial flow ;
- converting said flow of dihydrogen into a produced hydrogen-based flow by chemical reaction with a reacting agent from a reagent flow, said produced hydrogen-based flow comprising H₂ derivatives, preferably chosen between NH₂, CH₄ or CH₃OH, or methycyclohexane

the method comprising the following preliminary steps of:
   - measuring, in samples of the initial flow and of the reagent flow, ratios of stable isotopes of at least one element, to obtain feedstock isotope ratios ;
   - associating each feedstock isotope ratio with a unique identifier to form identification information and storing said identification information in a distant server,
the method further comprising a preliminary step of acquiring a partition coefficient specific to the generating step and a partition coefficient specific to the converting step, each partition coefficient being correlated to the variation of the stable isotopes during the generating step, respectively the converting step.

Depending on the origin of the feedstocks, the natural isotopic ratios of the initial and reagent flows will vary. The ratios will also vary during the generation and converting steps. The isotope content of the produced hydrogen-based flow will thus be a unique signature of its origin.

The method according to the invention allows a reliable certification of the origin of an hydrogen-based flow, based on isotope crossed mapping.

The unique identifier allows traceability of the hydrogen-based flow, making it possible to determine its origin, via the analysis of ratios of stable isotopes.

There is no need to add additional tracers in the hydrogen-based flow, preventing degradation of its quality, which would make it unsuitable for some applications, such as fuel cells.

The method according to the invention may comprise one or more of the following features, taken alone or in any technically feasible combination:
- the method further comprises a step of computing the identification information with the partition coefficients specific to the generating step and the converting step to obtain an expected profile of isotope ratios.
- the expected profile is defined by an electronic computer, this electronic computer comprising a model having in memory the partition coefficients specific to the generating and converting steps.
- the method further comprises the steps of measuring, in a sample of a purchased hydrogen-based flow, ratios of stables isotopes, then obtaining a measured profile of ratios of said isotopes; comparing the expected profile of isotope ratios with a request for authenticating, said request for authenticating comprising the measured profile; and based on the comparison, generating an authentication confirmation if the expected profile of isotope ratios matches the request for authenticating during an authenticating step.
- the ratios of stable isotopes measured during the measuring steps are chosen between D/H and/or one among ¹⁵N/¹⁴N, ¹⁸O/¹⁶O, ¹⁷O/¹⁶O, ¹⁴C/¹²C and ¹³C/¹²C.
- the flow of dihydrogen is generated by water electrolysis of a water flow, the preliminary step of acquiring the partition coefficient specific to the generating step comprising the following sub-steps of carrying out a calibration by adding known quantities of D₂O into water samples; for each sample, generating a dihydrogen sample by water electrolysis; measuring D/H ratio of each dihydrogen sample to obtain a table of isotopic partition coefficients.
- the produced hydrogen-based flow is chosen between NH₃, MeOH and CH₄.
- the feedstock isotope ratios are obtained by gas chromatography, notably by gas chromatography-isotope ratio-mass spectrometry.
- the ratios of stables isotopes in the purchased hydrogen-based flow are obtained by spectroscopy, preferably by high-resolution mass spectrometry.
- the method further comprises a step of emitting a certificate comprising the identification information and sending said certificate to an electronic receiving device.
- the step of sending said certificate to the electronic receiving device is carried out prior the step of computing the identification information with the partition coefficients.
- the method further comprises a step of emitting a certificate comprising the expected profile.
- the certificate is an electronic certificate.

The invention also relates to a computer program product comprising software instructions which, when executed by a processor, implement a method as described above.

The invention also relates to a system for certifying origin of a purchased hydrogen-based flow, the system being configured to carry out the method as described above, the system comprising:
- a measuring device for measuring ratios of stable isotopes in samples of the initial flow and of the reagent flow to obtain feedstock isotope ratios ;
- an identification device for associating each feedstock isotope ratio with a unique identifier to form identification information and storing said identification information in a distant server.

### BRIEF DESCRIPTION OF THE DRAWINGS

The invention and its advantages will be better understood upon reading the following description, which is given solely by way of non-limiting examples and which is made with reference to the appended drawings, in which:
- Figure 1 is a schematic diagram showing a system for certifying origin of a hydrogen-based flow according to a first embodiment of the invention.
- Figure 2 is a flowchart of a method for certifying origin of a purchased hydrogen-based flow, the method being performed by the system of Figure 1.
- Figure 3 is a schematic diagram showing a system for certifying origin of a hydrogen-based flow according to a second embodiment of the invention.
- Figure 4 is a flowchart of a method for certifying origin of a purchased hydrogen-based flow, the method being performed by the system of Figure 3.

### DETAILED DESCRIPTION OF THE INVENTION

Before describing the invention, it is to be understood that it is not limited to the details of construction or process steps set forth in the following description. It will be apparent to those skilled in the art having the benefit of the present disclosure that invention is capable of other embodiments and of being practiced or being carried out in various ways.

In the following description, the term "hydrogen-based flow" is used in reference to a flow of dihydrogen derivatives. Said dihydrogen derivatives are made from H₂ molecules that originate for example from water electrolysis, natural gas reforming or any other suitable processes and from other molecules, such as N₂,CO₂, CO, toluene, methytoluene, or any hydrocarbon or any hydrogen carrier (liquid organic hydrogen carriers or inorganic carriers).

In the following description, unless indicated otherwise, the ratio of A/B, also called A/B ratio, is to be understood as the ratio of the mass concentration of element A by the mass concentration of element B.

As used herein, the term "distant server" may refer to one or several computers able to provide a distant service such for one or several certification systems. The distant service can be accessible by a user via a terminal. In some embodiments, the distant server is able to store electronic certificates.

As used herein, the term "unique identifier" may refer to a number or a series of alphanumeric characters or any other forms of identification, making it possible to identify each initial and reagent flows, preferably in a unique way.

### FIRST EMBODIMENT OF THE INVENTION

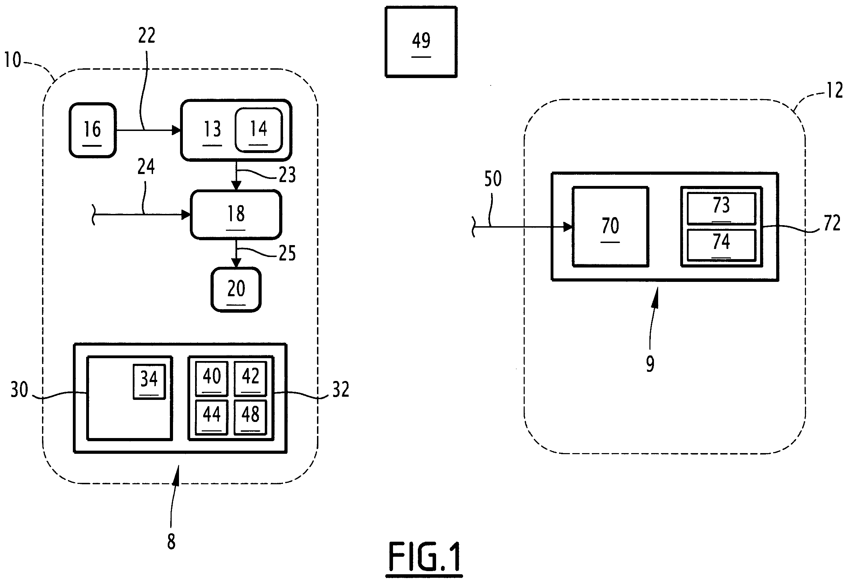

Referring to Figure 1, a system 8 for certifying origin of a hydrogen-based flow, also called certification system 8, according to a first embodiment of the invention will be first described.

Particularly, the system 8 is configured to be implemented in an installation 10 for producing a hydrogen-based flow, also called producing installation 10.

The hydrogen-based flow is intended to be transported to and received by a second installation 12, called purchasing installation 12. Said purchasing installation 12 may be for example a dehydrogenation unit, or a chemical plant.

As it will be described later, the purchasing installation 12 comprises a system 9 for verifying the origin of a hydrogen-based flow, also called verification system 9.

The producing installation 10 comprises a generation unit 13 configured to produce a produced flow of dihydrogen 23 from an initial flow 22.

For example, in the embodiment shown on Figure 1, the installation 10 is a electrochemical installation, notably for the generation of dihydrogen and of dioxygen from water electrolysis.

The generation unit 13 comprises an electrolysis unit 14 adapted for producing a dihydrogen flow 23 by water electrolysis and a balance of plant connected to the electrolysis unit 14, the balance of plant comprising various fluid handling components to manage the incoming fluids (in particular steam, electricity, air/nitrogen) and the outgoing fluids (hydrogen and oxygen).

The installation 10 comprises an electrolyte reservoir 16 fluidly connected to the electrolysis unit 14 and configured to supply the electrolysis unit 14 with electrolyte, which is the initial flow. The electrolyte reservoir 16 is for example a tank, a pipe system or any other enclosure suitable for the electrolyte.

Advantageously, the electrolyte is water obtained by seawater purification. The electrolyte reservoir 16 is for example connected to a desalinization assembly (not shown).

The installation 10 further comprises a conversion unit 18 and a storage unit 20.

The conversion unit 18 is configured to convert the produced flow of dihydrogen 23 into a flow of dihydrogen derivatives 25, also called produced hydrogen-based flow 25, by chemical reaction with a reagent flow 24.

For example, the conversion unit 18 is a synthesis module for the production of methanol and/or methanol derivatives from hydrogen generated in the electrolysis unit 14 and CO₂ of a stream of CO₂-rich gas, said CO₂-rich gas coming for example from combustion of fuel. The reagent flow is here the stream of CO₂-rich gas.

In a variant, the conversion unit 18 is a methanation module for the production of methane CH₄ from hydrogen generated in the electrolysis unit 14 and CO₂ of a stream of CO₂-rich gas (Sabatier process). The reagent flow is here the stream of CO₂-rich gas.

In a variant, the conversion unit 18 is a synthesis module for the production of ammonia from hydrogen generated in the electrolysis unit 14 and diazote N₂ of a stream of N₂, by implementation of the Haber-Bosch catalytic process. The reagent flow is here the stream of N₂.

In a variant, the conversion unit 18 is a hydrogenation module for the production of methyltoluene from hydrogen generated in the electrolysis unit 14 and methylcyclohexane of a stream of CO₂-rich gas (Sabatier process). The reagent flow is here the stream of methylclohexane liquid.

The storage unit 20 is configured to store the produced hydrogen-based flow 25, before its delivery to the purchasing installation 12.

The system 8 comprises a measuring device 30 and an identification device 32.

The measuring device 30 is configured to measure ratios of stable isotopes in samples of the initial flow 22 and of the reagent flow 24 to obtain feedstock isotope ratios, as it will be explained later.

Preferably, the measuring device 30 comprises a communication module 34 suitable to communicate the obtained measurements to the identification device 32.

For example, the measuring device 30 comprises a gas chromatographer, preferably a gas chromatography-isotope ratio-mass spectrometer.

The identification device 32 is configured to receive the measurements communicated by the measuring device 30 and to process them in order to form identification information.

According to the first embodiment, the identification device 32 comprises an association module 40, a computation module 42, a certificate emission module 44 and a transmission module 48.

The association module 40 is configured to receive isotope ratios from the communication module 34 of the measuring device 30. In complement or in a variant, the association module 40 is configured to receive isotope ratios entered manually, for example thanks to a manual input device.

The association module 40 is configured to associate each feedstock isotope ratio with a unique identifier, in order to form identification information.

The unique identifier associated with the initial flow 22 includes information about the origin of the hydrogen, in particular the hydrogen type and/or the hydrogen production method.

Hydrogen type is information indicating types of hydrogen, such as Gray hydrogen, Low Carbon hydrogen, and Green hydrogen.

Hydrogen production method is information indicating the process by which hydrogen is produced.

The unique identifier associated with the reagent flow 23 includes information about the origin of said reagent flow 23. For example, when the reagent flow 23 is CO₂, the unique identifier may include information on the process used to obtain said CO₂ flow. The unique identifier may include information preferably includes information on the full chain to be considered to produce the molecule. For example, the CO₂ flow may be obtained by CO₂-capture that is placed downstream of a chemical plant or power plant : the unique identifier includes information on the capture process itself and on the processes leading to the formation of CO₂.

Using each unique identifier, the association module 40 is able to form identification information for each flow.

The computation module 42 is configured to compute each identification information with partition coefficients specific to the process implemented by the generation unit 13 and the conversion unit 18, as it will be explained later. The computation module 42 is configured to generate an expected profile of isotope ratios based on said computation.

The certificate emission module 44 is configured to issue a certificate comprising the expected profile. Advantageously, the certificate is an electronic certificate certifying the origin of the produced hydrogen-based flow. The certificate may contain additional information, such as the date of the certificate issuance, the date of production of the hydrogen flow, the name of the producer of the hydrogen-based flow, the origin of the energy used to produce the hydrogen flow or the geographic location of the plant used to produce the hydrogen flow (e.g. GPS coordinates).

Advantageously, the information contained in the certificate is encrypted.

The transmission module 48 is configured to send the certificate to a distant server 49. For this purpose, the transmission module 48 is connected to the distant server 49 using for example a global communication network such as Internet.

The identification device 32 is typically an electronic computer provided with a memory and a microprocessor associated to said memory. The association module 40, the computation module 42, the certificate emission module 44 and the transmission module 48 are each realized in the form of a software, or a software brick, executable by the microprocessor.

Advantageously, the memory is able to store a model comprising the partition coefficients specific to the processes implemented by the generation unit 13 and the conversion unit 18. The processor is able to execute said model.

In a variant, the association module 40, the computation module 42, the certificate emission module 44 and the transmission module 48 are each realized in the form of a programmable logic component, such as an FPGA (Field Programmable Gate Array), or in the form of a dedicated integrated circuit, such as an ASIC (Application Specific Integrated Circuit).

When the identification device 32 is realized in the form of one or more software programs, i.e., in the form of a computer program, it is further adapted to be recorded on a computer-readable medium, not shown. The computer-readable medium is, for example, a medium capable of storing electronic instructions and of being coupled to a bus of a computer system. For example, the readable medium is an optical disk, a magneto-optical disk, a ROM memory, a RAM memory, any type of non-volatile memory (e.g. EPROM, EEPROM, FLASH, NVRAM), a magnetic card or an optical card. A computer program with software instructions is stored on the readable medium.

As mentioned above, the system 9 for verifying the origin of a hydrogen-based flow is located in the purchasing installation 12.

The system 9 comprises a measuring device 70 for measuring isotope ratios in a purchased hydrogen-based flow and an electronic-receiving device 72.

The electronic-receiving device 72 is configured to access to a certificate stored in the distant server 49.

Preferably, the electronic-receiving device 72 comprises a reception module 73 configured to receive the certificate and a comparison module 74 configured to compare the expected profile registered in said certificate and the measured profile obtained by the measuring device 70.

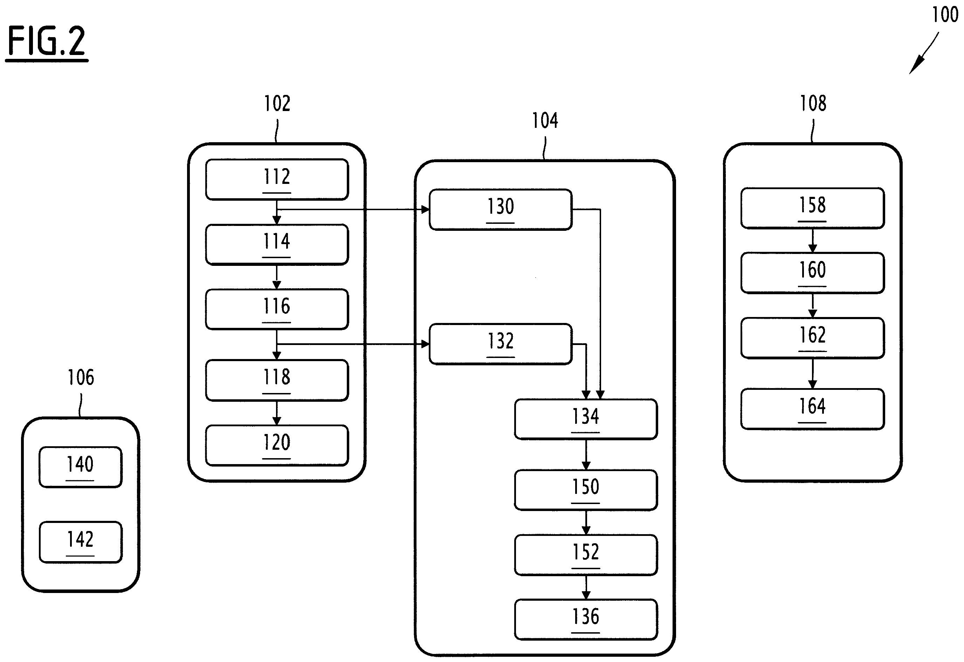

A method for certifying origin of a hydrogen-based flow, called also method 100, will now be explained in reference to Figure 2 presenting a flowchart of its steps. The method 100 is performed by the system 8 according to the first embodiment of the invention.

The method 100 comprises a production phase 102 and a certification phase 104. Advantageously, the method 100 further comprises a preliminary calibration phase 106 and a final authentication phase 108. Typically, the calibration, the production and the certification phases 106, 102, 104 are carried out by in the producing installation 10 and the authentication phase 108 is carried out in the purchasing installation 12.

The production phase 102 is typically carried out in the producing installation 10.

Initially, an initial flow 22 is provided to the generation unit 13 in an initial supplying step 112.

In the embodiment of Figure 2, the electrolyte is supplied into the electrolysis unit 14 of the installation 10.

A produced flow of dihydrogen 23 is then generated in the electrolysis unit 14 during a generating step 114, by water electrolysis. As known per se, electrolysis of water is the decomposition of water (H₂O) into dioxygen (O₂) and dihydrogen gas (H₂) due to an electric current being passed through the water.

The produced flow of dihydrogen 23 is then collected during a collecting step 116.

The produced flow of dihydrogen 23 is then converted into a produced hydrogen-based flow 25 during a converting step 118.

For example, the produced flow of dihydrogen 23 is combined with a flow of nitrogen to produce ammonia (Haber-Bosch process). In a variant, the produced flow of dihydrogen 23 is mixed with a flow a carbon dioxide or carbon monoxide to form methane CH₄ or methanol CH₃OH. In a variant, the produced flow of dihydrogen 23 is mixed with a flow of any hydrogen carrier, such as toluene or methyltoluene, to form hydrogenated hydrogen carriers.

Then, during a storing step 120, the produced flow of dihydrogen derivatives is stored in the storage unit 20, before being shipped to the purchasing installation 12. The produced flow of dihydrogen derivatives is typically stored in tanks or in underground reservoirs or directly into a tube-trailer or a ship.

The certification phase 104 is carried out at least partly simultaneously to the production phase 102.

The certification phase 104 comprises at least one measuring step 130, an associating step 134 and an information storing step 136.

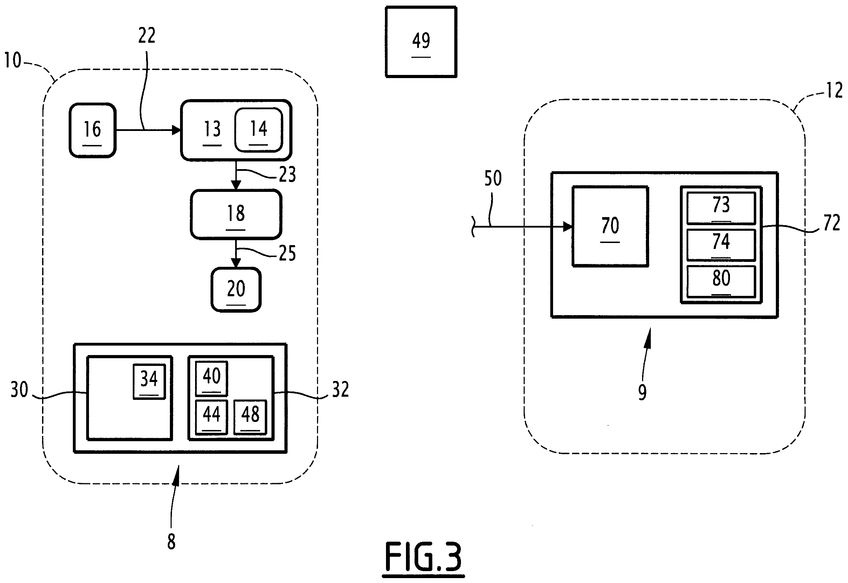

Advantageously, as shown in the embodiment of Figure 3, the certification phase 104 comprises a first measuring step 130, a second measuring step 132, an associating step 134 and an information storing step 136.

Prior to the generating step 114 and the converting step 118, ratios of stable isotopes of at least one element are measured in the initial flow 22 and in the reagent flow 24, to obtain feedstock isotope ratios.

The first measuring step 130 is carried out prior to the generating step 114. During this step, at least one ratio of stable isotopes of at least one element is measured in a sample of the initial flow 22.

In particular, according to the first embodiment, ratio of deuterium/hydrogen (D/H) is measured in the initial flow 22 of water prior to the generation of dihydrogen during the first measurement step 130. As known per se, water contains traces of deuterium, its concentration depending of the origin of the water.

The second measuring step 132 is carried out prior to the converting step 118. During this step 132, at least one ratio of stable isotopes of at least one element is measured in a sample of the reagent flow 24.

Depending on the process used in the conversion step 118, at least one ratio chosen among ¹⁵N/¹⁴N, ¹⁸O/¹⁶O, ¹⁷O/¹⁶O, ¹⁴C/¹²C or ¹³C/¹²C ratio is measured in the reagent flow 24 during the second measurement step 132.

For example, when the reagent flow 24 is a flow of nitrogen, the ratio of ¹⁵N/¹⁴N is measured in the reagent flow 24.

In a variant, when the reagent flow 24 is a carbon dioxide, at least one among the ratio of ¹⁸O/¹⁶O or ¹⁷O/¹⁶O and the ratio of ¹⁴C/¹²C or ¹³C/¹²C is measured in the reagent flow 24.

The ratios of stable isotopes measured during the measuring steps 130, 132 are called feedstock isotope ratios.

The measurements during steps 130, 132 are carried out by the measuring device 30.

Advantageously, the measurements during steps 130, 132 are carried out by gas chromatography, notably by gas chromatography-isotope ratio-mass spectrometry.

Then, the association module 40 of the identification device 32 associates each feedstock isotope ratio with a unique identifier during the associating step 134. As explained above, the association module 40 can determine the isotope ratios using for example data transmitted by the communication module 34 of the measuring device 30 or by manual input.

During the associating step 134, the D/H ratio of the initial flow 22 of water is associated with a unique identifier to form hydrogen-related identification information. Said identification information is then stored in the distant server 49 during the information storing step 136.

During the associating step 134, the isotope ratio measured in the reagent flow 24 during the second measurement step 132 is also associated with a unique identifier to form reactant-related identification information. Said identification information is then stored in the distant server 49 during the information storing step 136.

The association during step 134 is carried out automatically by the association module 40.

According to the first embodiment, the certification phase 104 further comprises a computation step 150 and a certificate emission step 152. Said steps 150, 152 are carried out between the associating step 134 and the information storing step 136.

During the computation step 150, each identification information is computed with partition coefficients specific to the generating step 114 and/or the converting step 118 to obtain an expected profile of isotope ratios.

Said partition coefficients have preferably been previously obtained during the preliminary calibration phase 106, which will be detailed later.

The expected profile of isotope ratios integrates an isotopic signature of several elements. In particular, the expected profile comprises the expected D/H ratio and at least one among the expected ¹⁵N/¹⁴N, ¹⁸O/¹⁶O, ¹⁷O/¹⁶O, ¹⁴C/¹²C and ¹³C/¹²C ratios, depending on the converting step 118.

Then, during the certificate emission step 152, a certificate comprising the expected profile is emitted by the certificate emission module 44.

The certificate is preferably an electronic certificate.

The certificate is then transmitted to and made available on the distant server 49. For this purpose, the transmission module 48 preferably uses a secure channel of the TLS type, such as an HTTPS channel which is encrypted and authenticated, or any blockchain based method.

The partition coefficients specific to the production phase 102 are acquired during the preliminary calibration phase 106.

The preliminary calibration phase 106 comprises a step 140 of acquiring the partition coefficient specific to the generating step 114 and a step 142 of acquiring the partition coefficient specific to the converting step 118

Each partition coefficient is correlated to the variation of the stable isotope during the generating step 114, respectively the converting step 118.

During step 140, known quantities of heavy water D₂O are added into a set of water samples to carry out a calibration, so that the ratio of D/H in the samples varies from 0% to 100% (molar), preferably from 0% to 10% (molar).

For each sample, a dihydrogen flow sample is generated by water electrolysis using the generation device 13 with the same parameters than the one used during the generating step 114.

The D/H ratio of each dihydrogen sample is then measured, for example by spectroscopy, preferably by high resolution mass spectrometry.

The isotopic partition coefficient specific to the generating step 114 is thus obtained based on the variation of the stable isotope ratio during said step 114. Advantageously, said isotopic partition coefficient is then recorded in the model housed in the memory of the identification device 32.

During step 142, known quantities of isotope of interest are added into a set of samples of reagent flow 24 to carry out a calibration.

For example, when the converting step 118 consists in the production of ammonia, known quantities of ¹⁵N are added into a set of N₂ samples to carry out a calibration.

When the conversion step 118 consists in the production of methane, known quantities of ¹⁴C or ¹³C are added into a set of CO₂ samples to carry out a calibration.

When the conversion step 118 consists in the production of methanol, known quantities of ¹⁸O or ¹⁷O or ¹⁴C or ¹³C are added into a set of CO₂ samples to carry out a calibration.

For each sample, a sample of dihydrogen derivative is generated using the conversion unit 18 with the same parameters than the one used during the converting step 118.

The isotope ratio of each sample of dihydrogen derivative is then measured to obtain the isotopic partition coefficient specific to the conversion step 118, based on the variation of the stable isotope ratio during said step 118. Advantageously, said isotopic partition coefficient is then recorded in the model housed in the memory of the identification device 32.

Advantageously, isotopic partition coefficient specific to the conversion step 118 is also calculated for the D/H ratio, said isotopic partition coefficient being then recorded in the model housed in the memory of the identification device 32.

The final authentication phase 108 is performed when one is intending to verify the origin of a hydrogen-based flow to be authenticated.

The final authentication phase 108 is preferably carried out in the purchasing installation 12.

First, a hydrogen-based flow 50, also called purchased hydrogen-based flow 50 or hydrogen-based flow to be authenticated 50 is obtained in an acquisition step 158.

During a final measuring step 160, isotope ratios of the purchased hydrogen-based flow 50 are measured, for example by using by spectroscopy, preferably high-resolution mass spectrometry.

During step 160, the D/H ratio and at least one among the ¹⁵N/¹⁴N, ¹⁸O/¹⁶O, ¹⁷O/¹⁶O, ¹⁴C/¹²C or ¹³C/¹²C ratios are measured.

A measured profile of ratios of said isotopes is thus obtained.

According to the first embodiment, the electronic-receiving device 72 then generates a request for receiving the certificate stored in the distant server 49. The certificate is received by the electronic-receiving device 72.

Advantageously, the distant server 49 is only accessible to authorized users.

Then, the measured profile is compared with the expected profile of isotope ratios during a comparing step 162.

If the expected profile of isotope ratios matches the measured profile, an authentication confirmation is then generated during an authenticating step 164.

If the expected profile of isotope ratios does not match the measured profile, no authentication confirmation is generated. Advantageously, a mismatch signal is then emitted during the authentication step 164.

By "matching", it is meant that the ratios of isotopes of the measured profile differ from the ratios of isotopes of the expected profile by no more than 10%, advantageously no more than 1.0%.

The method according to the first embodiment allows to verify the origin of a purchased hydrogen-based-flow in a secure and reliable manner.

The method according to the invention allows a reliable certification of the origin of an hydrogen-based flow, based on isotope crossed mapping.

The quality of the hydrogen-based flow is not altered, since no tracer has to be added.

Electronic certificates allow a secure data storage.

### SECOND EMBODIMENT OF THE INVENTION

Figure 3 shows a system 8 for certifying origin of a hydrogen-based flow according to a second embodiment of the invention.

According to this embodiment, the system 8 comprises the same components as those explained above. Thus, the same numerical references will be used to designate these components.

Unlike the first embodiment, the identification device 32 comprises an association module 40, a certificate emission module 44 and a transmission module 48. The identification device 32 is deprived of a computation module.

The certificate emission module 44 is configured to issue a certificate comprising the feedstock isotope ratios, instead of the expected profile.

In the second embodiment, the system 9 for verifying the origin of a hydrogen-based flow comprises a computation module 80, for example included in the electronic-receiving device 72.

The method performed by the system according to the second embodiment of the invention is similar to the method explained above, except the computation step 150.

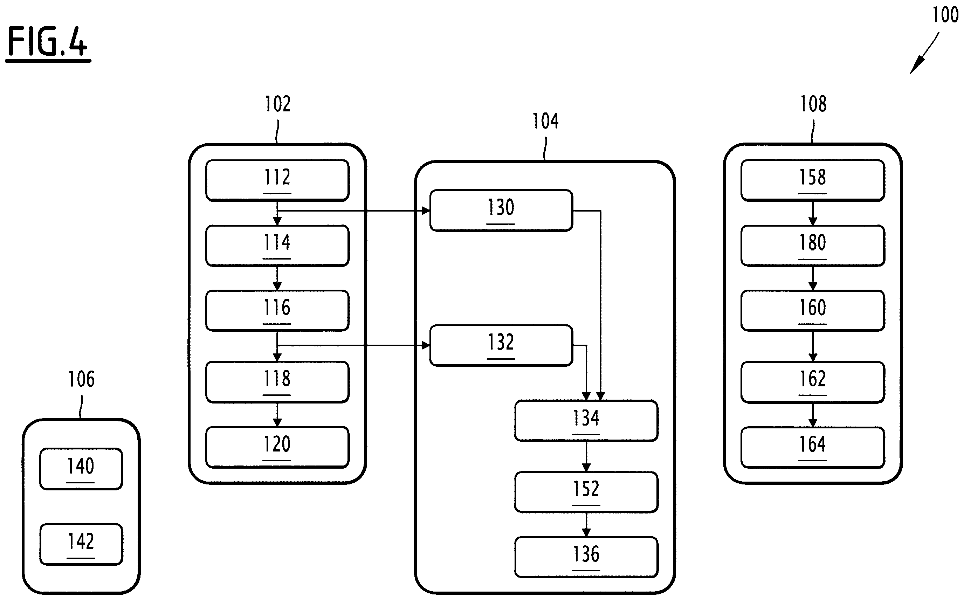

Figure 4 shows a flowchart of the steps of the method 100 performed by the system 8 according to the second embodiment of the invention

In this case, the computation of each identification information with the associated partition coefficient is performed in the purchasing installation 12, after reception of the certificate, during the final authentication phase 108. The expected profile is thus directly calculated in the purchasing installation 12 during a computating step 180, making it more secure and easier for an end user at the purchasing installation 12 if different providers produced the upstream feedstock for the hydrogen-based flow.

The partition coefficients may be stored in the distant server 49 or directly sent to the electronic-receiving device 72, for example at the time of the shipment of the hydrogen-based flow.

### OTHER EMBODIMENTS

One can conceive that many other embodiments of the invention are possible. Particularly, it will be clear for a person skilled in the art, that the modules of the systems 8, 9 can be arranged in different way.

According to a variant of the first and second embodiments, the comparing step 162 is performed directly in the distant server 49.

According to said variant, the electronic-receiving device 72 generates a request for authenticating comprising the measured profile and send it to the distant server 49.

Then, the measured profile is compared with the expected profile of isotope ratios stored in the certificate or with the expected profile measured directly in the distant server 49 based on the feedstock isotope ratios registered in the certificate.

The comparison is done distantly.

If the expected profile of isotope ratios matches the measured profile, an authentication confirmation is then generated and sent to the electronic-receiving device 72.

If the expected profile of isotope ratios does not match the measured profile, no authentication confirmation is generated and/or a mismatch signal is then transmitted to the electronic-receiving device 72.

In a variant, the flow of dihydrogen 23 is produced by a process different than water electrolysis during the generation step 114.

For example, the flow of dihydrogen 23 is produced by process chosen among natural gas reforming, methane pyrolysis, biomass pyrolysis and photoelectrocatalysis

The method performed by the system according to said variant of the invention is similar to the method explained above, except the generating step 114, the first measuring step 130 and the step 140 of acquiring the partition coefficient specific to the generating step 114.

## Claims

1. A method (100) for certifying origin of a purchased hydrogen-based flow, comprising the following steps:
- generating (114), notably by water electrolysis or natural gas reforming, a flow of dihydrogen (23) from an initial flow (22) ;
- converting (118) said flow of dihydrogen (23) into a produced hydrogen-based flow (25) by chemical reaction with a reacting agent from a reagent flow (24), said produced hydrogen-based flow (25) comprising H₂ derivatives, preferably chosen between NH₂, CH₄ or CH₃OH, or methylcyclohexane,
the method comprising the following preliminary steps of:
- measuring (130, 132), in samples of the initial flow (22) and of the reagent flow (23), ratios of stable isotopes of at least one element, to obtain feedstock isotope ratios ;
- associating (134) each feedstock isotope ratio with a unique identifier to form identification information and storing (136) said identification information in a distant server (49),
the method further comprising a preliminary step (140, 142) of acquiring a partition coefficient specific to the generating step (114) and a partition coefficient specific to the converting step (118), each partition coefficient being correlated to the variation of the stable isotopes during the generating step (114), respectively the converting step (118).

2. The method (100) according to claim 1, further comprising a step of computing (150; 180) the identification information with the partition coefficients specific to the generating step (114) and the converting step (118) to obtain an expected profile of isotope ratios.

3. The method (100) according to claim 2, wherein the expected profile is defined by an electronic computer, this electronic computer comprising a model having in memory the partition coefficients specific to the generating and converting steps (114; 118).

4. The method (100) according to claim 2 or 3, further comprising the steps of:
- measuring (160), in a sample of a purchased hydrogen-based flow, ratios of stables isotopes, then obtaining a measured profile of ratios of said isotopes;
- comparing (162) the expected profile of isotope ratios with a request for authenticating, said request for authenticating comprising the measured profile; and
- based on the comparison, generating an authentication confirmation if the expected profile of isotope ratios matches the request for authenticating during an authenticating step (164).

5. The method (100) according to any of the preceding claims, wherein the ratios of stable isotopes measured during the measuring steps (130, 132) are chosen between D/H and/or one among ¹⁵N/¹⁴N, ¹⁸O/¹⁶O, ¹⁷O/¹⁶O, ¹⁴C/¹²C and ¹³C/¹²C. .

6. The method (100) according to any of the preceding claims, wherein the flow of dihydrogen (23) is generated by water electrolysis of a water flow, the preliminary step of acquiring (140) the partition coefficient specific to the generating step (114) comprising the following sub-steps of:
- carrying out a calibration by adding known quantities of D₂O into water samples;
- for each sample, generating a dihydrogen sample by water electrolysis;
- measuring D/H ratio of each dihydrogen sample to obtain a table of isotopic partition coefficients.

7. The method (100) according to any of the preceding claims, wherein the produced hydrogen-based flow (25) is chosen between NH₃, MeOH and CH₄.

8. The method (100) according to any of the preceding claims, wherein the feedstock isotope ratios are obtained by gas chromatography, notably by gas chromatography-isotope ratio-mass spectrometry.

9. The method (100) according to any of the preceding claims, wherein the ratios of stables isotopes in the purchased hydrogen-based flow are obtained by spectroscopy, preferably by high-resolution mass spectrometry.

10. The method (100) according to any of the preceding claims, further comprising a step (152) of emitting a certificate comprising the identification information and sending said certificate to an electronic receiving device (72).

11. The method (100) according to claim 10 in combination with any of claim 2 to 4, wherein the step of sending said certificate to the electronic receiving device (72) is carried out prior the step of computing (180) the identification information with the partition coefficients.

12. The method (100) according to any of claims 1 to 9, further comprising a step (152) of emitting a certificate comprising the expected profile.

13. The method (100) according to any of claims 10 to 12, wherein the certificate is an electronic certificate.

14. A computer program product comprising software instructions which, when executed by a processor, implement a method (100) according to any of the preceding claims.

15. A system (8) for certifying origin of a purchased hydrogen-based flow, the system (8) being configured to carry out the method (100) according to any one of claims 1 to 13, the system (8) comprising :
- a measuring device (30) for measuring ratios of stable isotopes in samples of the initial flow (22) and of the reagent flow (24) to obtain feedstock isotope ratios ;
- an identification device (32) for associating each feedstock isotope ratio with a unique identifier to form identification information and storing said identification information in a distant server (49).
